Europäisches Patentamt

European Patent Office

Office européen des brevets

(19)

(11) Veröffentlichungsnummer : **0 456 033 B1**

(12) **EUROPÄISCHE PATENTSCHRIFT**

(45) Veröffentlichungstag der Patentschrift :
21.04.93 Patentblatt 93/16

(51) Int. Cl.⁵ : **C12N 9/24,** C12N 1/14,
C12P 19/04

(21) Anmeldenummer : **91106502.7**

(22) Anmeldetag : **23.04.91**

(54) **Verfahren zur Herstellung von Xylanase.**

(30) Priorität : **08.05.90 AT 1030/90**

(43) Veröffentlichungstag der Anmeldung :
**13.11.91 Patentblatt 91/46**

(45) Bekanntmachung des Hinweises auf die
Patenterteilung :
**21.04.93 Patentblatt 93/16**

(84) Benannte Vertragsstaaten :
**AT BE CH DE DK ES FR GB GR IT LI LU NL SE**

(56) Entgegenhaltungen :
**CHEMICAL ABSTRACTS, Band 71, Nr. 19, 10.
November 1968, Seite 108, Zusammenfassung
Nr. 88662s, Columbus, Ohio, US; V. BILAI et
al.: "Xylanase activity of some soil hyphal
fungi", & EKSP. MIKOL. 1968, 8-14
CHEMICAL ABSTRACTS, Band 92, Nr. 5, 4.
Februar 1980, Seite 399, Zusammenfassung
Nr. 37452z, Columbus, Ohio, US; A.Y. STRIZ-
HEVSKAYA: "Enzymic hydrolysis of xylans",
& MIKROBIOL. ZH. (KIEV) 1979, 41(5), 509-16
BIOTECHNOLOGY LETTERS, Band 9, Nr. 5,
1987, Seiten 353-356, Kew, GB; W. GRAJEK:
"Production of D-xylanases by thermophilic
fungi using different methods of culture"
J. FERMENT. TECHNOL., Band 62, Nr. 5, 1984,
Seiten 415-420, Suita, JP; V. KITPREECHAVA-
NICH et al.: "Purification and properties of
endo-1,4-beta-xylanase from Humicola lanuginosa"**

(56) Entgegenhaltungen :
**BIOTECHOLOGY LETTERS, Band 10, Nr. 12,
1988, Seiten 907-912, Kew, GB; D.J. SENIOR et
al.: "Selective solubilization of xylan in pulp
using a purified xylanase from Trichoderma
Harzianum"
ARCH. BIOCHEM. AND BIOPHYSICS, Band
276, Nr. 2, 1. Februar 1990, Seiten 546-553,
New York, US; L. ANAND et al.: "Purification
and properties of xylanase from the thermophilic Fungus, Humicola lanuginosa (Griffon
and Maublanc) Bunce"**

(73) Patentinhaber : **VOEST-ALPINE
Industrieanlagenbau GmbH
Turmstrasse 44
A-4020 Linz (AT)**

(72) Erfinder : **Wizani, Wolfgang, Dipl.-Ing.
Grillparzerstrasse 3
A-4400 Steyr (AT)**
Erfinder : **Esterbauer, Hermann, Dr.
Ziegelstrasse 21 W
A-8045 Graz (AT)**
Erfinder : **Steiner, Walter, Dr.
Im Hoffeld 1
A-8046 Graz (AT)**
Erfinder : **Gomes, Joseph, Dr.
113/2 East Rejabazar
Dhaka 1215 (BD)**

(74) Vertreter : **Kunz, Ekkehard, Dr.
Chemie Holding AG Patentwesen St.
Peter-Strasse 25
A-4021 Linz (AT)**

EP 0 456 033 B1

## Beschreibung

Die Erfindung betrifft ein Verfahren zur Herstellung von Xylanase, sowie den Mikroorganismus Thermomyces lanuginosus DSM 5826 und dessen Verwendung.

Der Abbau von Hemicellulose, die bei Einjahrespflanzen oder Laubhölzern vorwiegend aus Xylan besteht, ist ein in der Zellstofferzeugung notwendiger Schritt. Dieser Abbau kann entweder auf chemischem Weg, beispielsweise durch alkalische Heißextraktion oder auf enzymatischem Weg durch Behandlung mit substratspezifischen Enzymen, im speziellen mit Xylanasen erfolgen. Durch die enzymatische Behandlung des ungebleichten oder halbgebleichten Zellstoffs mit Xylanasen werden Hemicellulosebindungen aufgebrochen und Xylan abgebaut. Es können dazu jedoch nur reine Xylanasen eingesetzt werden, die keine Verunreinigungen an Cellulase enthalten, da sonst auch Cellulose gespalten und abgebaut wird, was äußerst unerwünscht ist.

Xylanasen, welche im Nährmedium vorhandene xylanhältige Rohstoffe abbauen und als C-Quelle verwerten, werden unter anderem von einer Reihe mesophiler und thermophiler Mikroorganismen gebildet. In Abhängigkeit von den weiteren im Nährmedium vorhandenen Rohstoffen, vorwiegend Cellulose werden aber auch die für dieses Substrat spezifischen Cellulasen gebildet. Um exo- und endocellulasefreie Xylanase zu erhalten, muß die Xylanase von den gebildeten Cellulasen in einem aufwendigen Verfahren getrennt und gereinigt werden. Um die Bildung von Cellulasen bei der Fermentation zu verringern, können die Mikroorganismen auch auf gereinigtem Xylan kultiviert werden. In D. J. Senior et al., Biotechnology Letters, Vol 10, No. 12, p. 907 - 912 (1988) wird die Verwendung von Xylanasen, die aus auf gereinigtem Xylan kultiviertem Trichoderma harzianum erhalten wurden, zum selektiven Abbau von Xylan beschrieben.

Die Gewinnung von Xylanase durch Kultivierung auf hochreinem Xylan kommt aber wegen der hohen Rohstoffkosten für die meisten technischen Anwendungen nicht in Betracht.

Es wurde nun unerwarteterweise gefunden, daß Xylanase mit überraschend hoher Aktivität produziert wird, wenn ein Pilz in einem Nährmedium, das Maiskolben enthält, kultiviert wird, wobei die entstandene Xylanase keine oder nur sehr geringe exo- und endo-Cellulaseaktivitäten aufweist.

Gegenstand der Erfindung ist daher ein Verfahren zur Herstellung von Xylanase, das dadurch gekennzeichnet ist, daß ein Pilz in einem Nährmedium kultiviert wird, das Maiskolben enthält.

Erfindungsgemäß wird Xylanase durch Kultivieren eines Pilzes in einem Nährmedium hergestellt, das Maiskolben enthält.

Unter Xylanase sollen dabei nur solche Xylanasen verstanden werden, die keine oder nur sehr geringe Aktivitäten an exo- oder endo-Cellulasen aufweisen.

Die Pilze, die dabei einsetzbar sind, sind solche Pilze, die im erfindungsgemäßen Verfahren hohe Xylanaseaktivitäten produzieren können. Ein Beispiel für solche Pilze ist Thermomyces lanuginosus (früher Humicola lanuginosa). Ein Stamm der Gattung Monylialis wurde aus einem Juteabfallhaufen einer Jutefabrik in Bangladesh, in der Jutefasern mit einer Ölemulsion behandelt werden, isoliert. Die Temperatur im Juteabfallhaufen betrug 65 bis 70°C. Dieser Stamm wurde bei der Deutschen Sammlung von Mikroorganismen und Zellkulturen unter DSM 5826 hinterlegt. Dieser Stamm ist besonders geeignet, in einem Nährmedium, das Maiskolben enthält, Xylanase mit überraschend hoher Aktivität zu produzieren.

Thermomyces lanuginosus DSM 5826 ist neu und ebenfalls Gegenstand der Erfindung.

Das Nährmedium, in dem der Pilz kultiviert wird, enthält neben den zum Wachstum nötigen Nährstoffen und Spurenelementen Maiskolben. Die Maiskolben können als solche oder gemahlen und gegebenenfalls durch Erhitzen auf 110 bis 160°C sterilisiert oder durch Vorbehandlung beispielsweise mit überhitztem Wasserdampf eingesetzt werden. Besonders hohe Xylanaseaktivitäten werden unerwarteterweise erreicht, wenn die Maiskolben vor ihrer Verwendung grob gemahlen werden. Werden die Maiskolben geschrotet oder fein gemahlen, sind die Ergebnisse zwar immer noch überraschend gut, die Verwendung von grob gemahlenen Maiskolben zeigte jedoch überragende Ergebnisse.

Geeignete Stickstoffquellen sind beispielsweise Fleischpepton, Fischpepton, Harnstoff, Ammoniumsulfat, Malzextrakt, Fleischextrakt, Sojamehl, Hefeextrakt, u. dgl, anorganische Salze beispielsweise Kaliumhydrogensulfat, Kaliumdihydrogenphosphat, Dinatriumhydrogenphosphat, Eisensulfat, Calciumchlorid, Magnesiumsulfat u. dgl..Um die Abgabe der Xylanase in das Nährmedium zu beschleunigen, kann es vorteilhaft sein, oberflächenaktive Substanzen zuzusetzen. Es werden üblicherweise nicht ionische oberflächenaktive Substanzen, beispielsweise Tween 40, Tween 60 oder Tween 80 in einer Menge von 0,05 bis 0,5 Gew.% bezogen auf die Gesamtmenge des Mediums eingesetzt. Dem Medium können gegebenenfalls auch Spurenelemente, beispielsweise seltene Metalle wie $Mn^{2+}$, $Zn^{2+}$, $Fe^{2+}$ oder Vitamine zugegeben werden.

Das Medium wird gegebenenfalls vorteilhafterweise mit Ammoniak bzw. Phosphorsäure auf einen pH-Wert von 5,0 bis 8,0, bevorzugt auf 6,0 bis 7,0 eingestellt.

Der Pilz wird im Nährmedium bei einer Temperatur von etwa 30 bis 70°C, bevorzugt bei 40 bis 60°C, besonders bevorzugt bei 45 bis 55°C kultiviert. Ein Konstanthalten des zu Beginn der Fermentation eingestellten pH-Wertes ist nicht nötig. Er kann jedoch gegebenenfalls durch Zudosieren von beispielsweise Ammoniak bzw. Phosphorsäure auch konstant gehalten werden.

Nach Beendigung der Fermentation kann die Xylanase auf übliche Weise aus der Fermentationsbrühe isoliert werden. Dazu werden beispielsweise Pilzmycel, Sporen und verbleibende ungelöste Stoffe durch Zentrifigieren oder Filtrieren abgetrennt. Die weitere Reinigung des Enzyms kann auf übliche Weise, beispielsweise durch Aussalzen mit Ammoniumsulfat oder durch Lösungsmittelfällung mit Aceton, Alkohol u. dgl. erfolgen. Das so erhaltene Rohenzym kann gegebenenfalls weiter, beispielsweise durch Gelfiltration, Ionenaustauschchromatographie, Gelelektrophorese u. dgl. gereinigt werden.

Die erfindungsgemäß hergestellte Xylanase weist eine hohe Xylanaseakivität auf. Es hat sich herausgestellt, daß bei Verwendung von Maiskolben gegenüber anderen unbehandelten Rohstoffen wie Gerstenspelzen, Weizenstroh, Weizenkleie, gemahlene Buchenrinde, Luzernenmehl, Rotkleegrasmehl, Sojaöl als C-Quelle überraschend hohe Xylanaseaktivitäten erreicht werden. Beim Einsatz grobmahlener Maiskolben erhöht sich die Aktivität der gebildeten Xylanase noch um ein vielfaches.

Die erfindungsgemäß hergestellte Xylanase enthält nur geringe oder keine exo- und endo-Cellulaseaktivitäten. Die von <u>Thermomyces lanuginosus DSM 5826</u> hergestellte Xylanase erwies sich in mehreren Tests als exo- und endo-cellulasefrei.

Dabei kann <u>Thermomyces lanuginosus DSM 5826</u> diese Xylanase nicht nur in einem Nährmedium produzieren, das Maiskolben enthält, sondern auch in einem Nährmedium, das anstatt Maiskolben andere feste oder gelöste xylanhaltige Kohlenstoffquellen wie z.B. Gerstenspelzen, gemahlenes Weizenstroh, ungebleichten Zellstoff oder Xylan selbst enthält. Überraschend hohe Xylanaseaktivitäten werden jedoch mit Maiskolben im Nährmedium erhalten.

Die erfindungsgemäß hergestellte exo- und endocellulasefreie Xylanase wies nach Ethanolfällung und Gefriertrocknung folgende Eigenschaften auf:

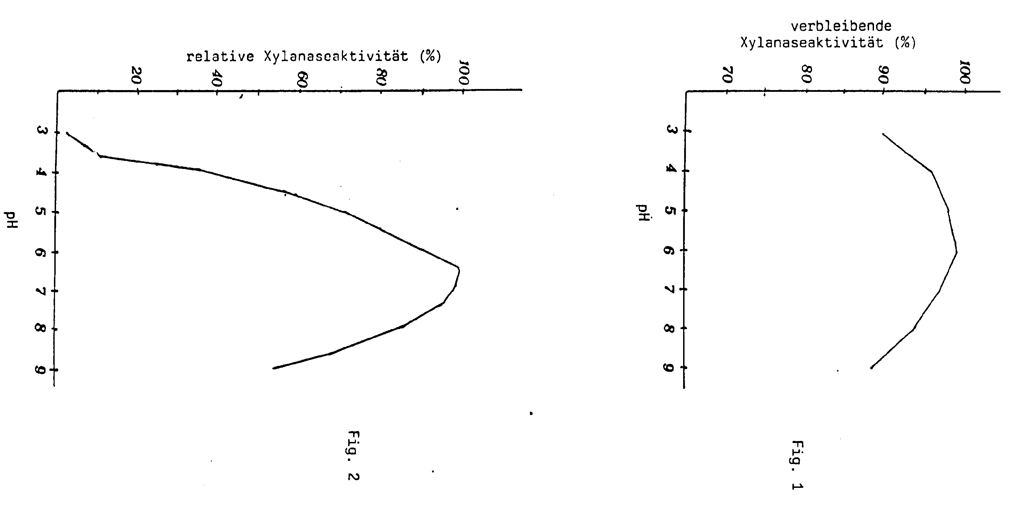

a) pH-Stabilität (Fig. 1)

Das Enzym wurde in Pufferlösungen bei verschiedenen pH-Werten 66 Stunden bei 20°C inkubiert. Die Aktivität aller Proben wurde mit 1 % Hemicellulose bei pH 4,8 wie folgt bestimmt: 1 ml 1 % Substratlösung in Na-Citratpuffer (pH 4,8), (Xylan aus Haferspelzen; Sigma X-0627;) wurden 2 min bei 50°C und nach Zufügen von 0,5 ml Enzymlösung weitere 15 min bei 50°C inkubiert. Anschließend wurden 3ml Dinitrosalicylsäure-Reagens (wie FPU-Test nach IUPAC) und 0,5 ml 2,5 N NaOH zugemischt und 5 min im kochenden Wasserbad erhitzt. Anschließend wurde in einem kalten Wasserbad rasch abgekühlt und die Extinktion bei 540 nm gegen den Leerwert (Citratpuffer) gemessen. Von diesen Wert muß die Extinktion des Enzyms (0,5 ml Enzym + 1,0 ml Citratpuffer) und der Substratlösung (1 ml 1 % Substratlösung in Citratpuffer) abgezogen werden. Die Eichkurve wurde mit 1,0 ml Citratpuffer und 0,5 ml Standardlösung (enthaltend 0,5 bis 1,5 mg Xylose/ml) erstellt.

Berechnung der Xylanaseaktivität:

XU/ml = mg reduzierender Zucker (als Xylose/Test) x 0,888

In einem pH-Bereich von 5,0 bis 7,0 wurden 97 bis 100 % der ursprünglichen Aktivität erhalten.

b) Optimaler pH-Wert (Fig. 2)

Die Enzymaktivität wurde durch Inkubieren (15 min, 50°C) mit 1 % Hemicellulose-Suspension in Citratpuffer (pH 3,0 bis 6,5), TrisHCl-Puffer (pH 7,0 bis 9,0) und Phosphatpuffer (pH 6,5 bis 8,0), ermittelt.

Optimaler pH-Bereich: 6,0 bis 7,5

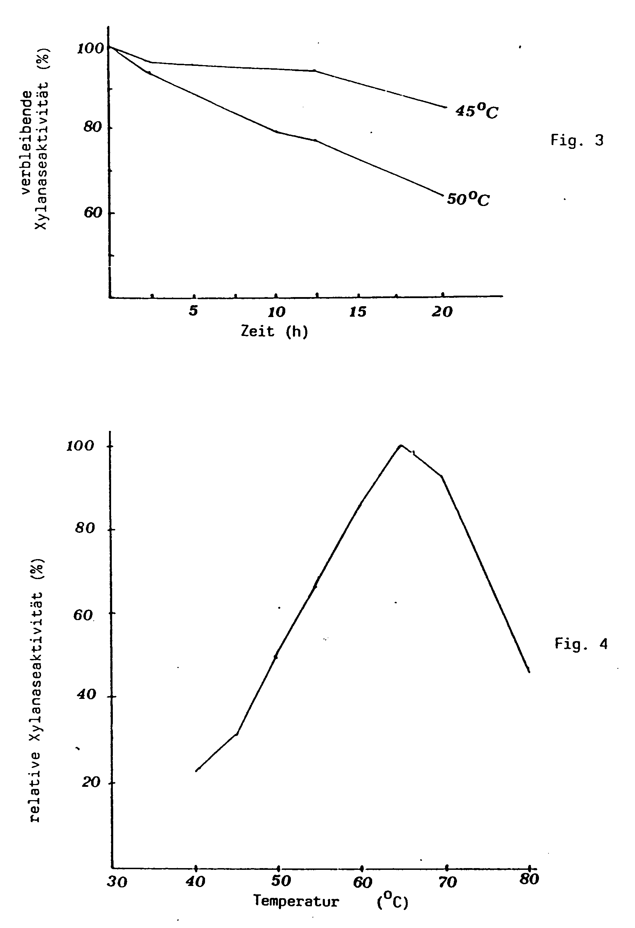

c) Thermostabilität (Fig. 3)

Die Enzymlösung wurde in 0,05 molaren Citratpuffer (pH 4,8) bei Temperaturen von 45 bis 60°C 0 bis 72 Stunden inkubiert. Die Aktivität des Enzyms wurde mit 1 % Hemicellulose bei 50°C gemessen.

Nach 20 Stunden wurden bei einer Temperatur von 45°C 93 % und bei einer Temperatur von 50°C 65 % der ursprünglichen Aktivität gemessen.

d) Optimale Temperatur (Fig. 4)

Die Enzymaktivität wurde durch Inkubieren mit einer 1 % Hemicellulosesuspension in 0,05 molaren Citratpuffer bei pH 4,8 15 min bestimmt.

Optimale Temperatur: 65°C

e) Gehalt einer Enzymlösung von 385 XU/ml an exo- und endo-Cellulasen und an β-Glucosidase:

i) exo- und endo-Cellulasen:

Im FPU-Test nach IUPAC wurde keine Cellulaseaktivität nachgewiesen.

Ferner wurde ein Dialyseschlauch (regenerierte Cellulose) 72 Stunden mit dem Enzym in einem wäßrigen Medium behandelt, und das Medium auf die Anwesenheit von Glucose geprüft, wobei keine Glucose gefunden wurde. Cellulasehältige Enzyme lösen den Dialyseschlauch dagegen binnen weniger Stunden.

Weiterhin wurde im Verlauf einer Langzeitinkubation des Enzyms auf Zellstoff bei einem pH Wert von 6,5 die Art der freigesetzten, reduzierenden Zucker bestimmt. Die Messungen erfolgten dabei nach 3, 7, 19 und 163 Stunden. Dabei zeigte sich, daß in allen Fällen nur Xylose, Xylobiosen und Xylotriosen, aber keine Glucose freigesetzt worden waren. Mit Hilfe einer Replikatechnik mit OBR-Hydroxyethylcellulose-gefärbten Agargel wurde auf endo-Cellulaseaktivitäten geprüft, wobei keine Aktivität aufgefunden wurde. Das Enzym war gemäß aller durchgeführter Versuche und Tests völlig frei von Cellulasen.

Die erfindungsgemäße Xylanase wies bei einer Nachweisgrenze von 0,1 Unit/ml auch keine Aktivität an Carboxymethylcellulase (endo-ß-1,4-Cellulase) auf und ist daher auch bei der Viskoseherstellung einsetzbar.

ii) ß-Glucosidase:

0,6 ml 0,05 molarer Natriumcitratpuffer (pH 4,8) und 0,3 ml Enzymlösung wurden 2 min bei 50°C vorinkubiert. Anschließend wurden 0,3 ml p-Nitrophenyl-β-D-Glucosid (4 mg/ml Natriumcitratpuffer) zugegeben und 10 min bei 50°C inkubiert. Die Reaktion wurde durch Zugabe von 2,4 ml 1 molarer $Na_2CO_3$-Lösung gestoppt und die Extinktion bei 405 mm gegen den Leerwert gemessen. Die Aktivität an ß-Glucosidase wurde wie folgt berechnet:

$$IU/ml = \frac{E \cdot 12}{18,5 \cdot x \cdot t}$$

E ... Extinktion

t ... Reaktionszeit (Minuten)

Die erfindungsgemäße Xylanase wies eine ß-Glucosidase-Aktivität von 0,2 bis 0,9 IU/ml auf.

Der Gehalt an β-Glucosidase, die Cellobiose in Glucose spaltet, hat keinen Einfluß auf das Verhalten der erfindungsgemäßen Xylanase in der Zellstoffherstellung, da aufgrund der Abwesenheit von exo- und endo-Cellulasen keine Abbauprodukte der Cellulose entstehen, die durch ß-Glucosidase angreifbar sind.

f) Ferner wurde eine beta-Xylosidase-, Arabinosidase-, Acetylesterase-, Acetylxylanesterase- und Mannanaseaktivität von je $\leqq$ 100 Units/l festgestellt.

g) Der Anteil des löslichen Proteins in der Enzymmischung betrug 800 mg/l, das Molekulargewicht des Hauptproteins 24 bis 25 kDa und der isoelektrische Punkt des Hauptproteins 4,1.

h) Das gereinigte Enzym wurde enzymatisch zu 11 Peptiden hydrolisiert. 4 Peptide mit 8, 16, 5 bzw. 12 Aminosäuren wurden sequenziert. Dabei erwies sich die untersuchte Xylanase als N-terminal blockiert und konnte daher vom N-terminalen Ende nicht ansequenziert werden, während eine Xylanase die aus Humicola lanuginosa sp. (= Thermomyces lanuginosus) gemäß Anand et al., Arch. Biochem. Biophys. 276, 546-553 (1990) isoliert worden war, als N-terminale Aminosäure Arginin enthielt.

Die erfindungsgemäß hergestellte Xylanase dient zur enzymatischen Behandlung von xylan- und lignocellulosehältigen pflanzlichen Rohstoffen und Faserstoffen aus solchen Rohstoffen und kann z.B. vorteilhaft zur Bleiche, zum Deinken, zur Veredelung des Zellstoffes bei der Viskoseherstellung oder für eine andere Vorbehandlung, wie z.B. Entfernung von Xylan vor dem Aufschluß eingesetzt werden.

Unter xylan- und lignozellulosehältigen pflanzlichen Rohstoffen sind Rohstoffe aus Laub- und Nadelhölzern, Einjahrespflanzen, z.B. Flachs, Stroh, Bagasse, Kenaf, Schilf, Elefantengras usw. aber auch Faserstoffe aus pflanzlichen Rohstoffen, wie gebleichter, halbgebleichter oder ungebleichter Zellstoff oder Altpapier zu verstehen.

Für die Anwendung muß die erfindungsgemäß hergestellte Xylanase nicht gereinigt werden, es genügt die Abtrennung des festen Nährmediums. Nach Abtrennung des festen Nährmaterials kann die Fermentationsbrühe als solche direkt zur Behandlung von Faserstoffen aus pflanzlichen Rohstoffen eingesetzt werden.

Beim Einsatz in der Zellstoffindustrie kann sowohl ungebleichter als auch halbgebleichter als auch gebleichter Zellstoff mit der erfindungsgemäß hergestellten Xylanase behandelt werden. Durch die Behandlung des Zellstoffs werden Hemicellulose-Lignin-bindungen aufgebrochen. Im anschließenden Bleichverfahren wird daher weniger Bleichmittel verbraucht. Bei der Behandlung des halbgebleichten Zellstoffs wird nach der Vorbleiche verbliebenes Xylan abgebaut, wodurch reinere und hellere Pulpen entstehen. Die Wirkung der Enzymbehandlung wird mit Hilfe der Kappa-Zahl (K), die den Gehalt an oxidierbarer Substanz, also zum Beispiel an Lignin angibt, bestimmt. Im gebleichten Zellstoff wird durch die Xylanasebehandlung ein höherer alpha-Cellulosegehalt erreicht.

Beispiel 1:

300 ml Medium, bestehend aus 9 g gemahlenen und getrockneten Maiskolben, 3,3 g Fleischpepton, 0,6 g $(NH_4)_2$ $SO_4$, 0,45 g Harnstoff, 0,09 g $MgSO_4.7H_2O$, 0,09 g $CaCl_2.2H_2O$, 4,5 g $KH_2PO_4$, 0,3 ml Tween 80, 0,3 ml $S_1$ (1,6 g/l $MnSO_4$ . H2O; 3,45 g/l $ZnSO_4$ . $7H_2O$; 2,0 g/l $CaCl_2$ . $6H_2O$) und 0,3 ml $S_2$ (5g/l $FeSO_4$ . $7H_2O$) wurden auf einen pH-Wert von 6 gestellt und in einem Schüttelkolben 60 min bei 128°C autoklaviert. Thermomyces lanuginosus DSM 5826-Vorkultur wurde in dieses Medium inokuliert und 4,5 Tage bei 50°C unter Schütteln (140 rpm) kultiviert.
Nach 4,5 Tagen wurde filtriert und die Aktivität an Xylanase (XU/ml), ß-Glucosidase, (IU/ml) Carboxymethylcellulase (CMC-ase/ml) und exo-Glucanase (FPU/ml) bestimmt.

| | |
|---|---|
| XU/ml | 389,7 |
| nach 3 Tagen Lagerung bei 4 °C | 395,0 |
| IU/ml | 0,29 |
| FPU/ml | nicht nachweisbar |
| CMC-ase/ml | nicht nachweisbar |

Beispiel 2:

Maiskolben wurden
    a) geschrotet (Größe der Maiskolbenstücke größer als 10 mm
    b) grob gemahlen (Größe der Maiskolbenstücke zwischen 3 und 10 mm)
    c) fein gemahlen (Größe der Maiskolbenstücke kleiner als 3 mm)
Es wurden jeweils 1000 ml eines Mediums, bestehend aus 28,6 g Hefeextrakt, 4,23 g $(NH_4)_2$ $SO_4$, 10 g $KH_2PO_4$, 0,3 g $FeSO_4.7H_2O$, 0,3 g $MgSO_4$ . $7H_2O$ und 0,3 g $CaCl_2.2H_2O$ und 25 g a) geschroteten, b) grob gemahlenen und c) fein gemahlenen, getrockneten Maiskolben hergestellt, auf einen pH Wert von 6,5 gestellt und in einem Schüttelkolben 25 Minuten bei 121°C autoklaviert.
Thermomyces lanuginosus DSM 5826-Vorkultur wurde in diese Medien inokuliert und bei 50°C unter Schütteln kultiviert.
Nach 5 Tagen wurde filtriert und die Aktivität an Xylanase (XU/ml) in den verschiedenen Medien bestimmt:

| Maiskolben | XU/ml | XU/ml nach 1 Tag Lagerung bei 4°C |
|---|---|---|
| geschrotet | 764 | 881 |
| grob gemahlen | 1601 | 1526 |
| fein gemahlen | 388 | 423 |

Beispiel 3:

In dem in Beispiel 2 beschriebenen Medium und auf die dort beschriebene Art und Weise, aber unter Verwendung anderer C-Quellen, wurden folgende Xylanaseaktivitäten ermittelt:

| C-Quelle | Xylanase-Aktivität (XU/ml) |
|---|---|
| Maiskolben (50 % fein gemahlen, 50 % grob gemahlen) | 477 |
| Weizenstroh | 232 |
| Weizenkleie | 186 |
| Gerstenspelzen | 61 |
| Ulva rifide (Alge) | 28 |
| Luzernenmehl | 20 |
| Rotkleegrasmehl | 14 |
| Buchenrinde gemahlen | 8 |
| Stärke löslich | 4 |
| Sojaöl | 4 |

Beispiel 4:

Thermomyces lanuginosus DSM 5826 wurde entsprechend Beispiel 1 kultiviert, wobei statt gemahlene Maiskolben 9 g gemahlenes Weizenstroh eingesetzt wurde.

| XU/ml | 104,3 |
|---|---|
| nach 3 Tagen Lagerung bei 4 °C | 123,9 |
| IU/ml | 0,51 |
| FPU/ml | nicht nachweisbar |
| CMC-ase/ml | nicht nachweisbar |

Beispiel 5:

Thermomyces lanuginosus DSM 5826 wurde entsprechend Beispiel 1 kultiviert, wobei statt gemahlene Maiskolben Gerstenspelzen eingesetzt wurden.

| XU/ml | 222,9 |
|---|---|
| nach 3 Tagen Lagerung bei 4 °C | 196,2 |
| IU/ml | 0,60 |
| FPU/ml | nicht nachweisbar |
| CMC-ase/ml | nicht nachweisbar |

Beispiel 6:

Thermomyces lanuginosus DSM 5826 wurde entsprechend Beispiel 1 kultiviert, wobei statt gemahlenen Maiskolben 9 g Hemicellulose (Xylan) eingesetzt wurde.

| XU/ml | 151,55 |
|---|---|
| IU/ml | nicht nachweisbar |
| FPU/ml | nicht nachweisbar |
| CMC-ase/ml | nicht nachweisbar |

Beispiel 7:

Behandlung von Sulfat-Zellstoff

Getrockneter Sulfatzellstoff aus Ruzomberok (Hartholz) wurde mit Wasser 1,5 Stunden auf 45°C erwärmt und geschüttelt. Anschließend wurde Xylanase, hergestellt analog Beispiel 1 zugegeben und bei 230 rpm geschüttelt.

```
a)   30 g Fermentationsbrühe     b) 90 g Fermentationsbrühe
     220 g Zellstoff (K=19,97)      220 g Zellstoff (K= 19,97)
     350 g Wasser                   290 g Wasser
     pro Gramm Faser wurden         pro Gramm Faser wurden
     100 XU Enzym zugegeben         300 XU Enzym zugegeben
```

Nach dem Abfiltrieren des Zellstoffs wurde die Kappa-Zahl bestimmt:

```
                              a)          b)
          Kappa-Zahl        17,45       13,39
```

## Patentansprüche

1. Verfahren zur Herstellung von Xylanase, dadurch gekennzeichnet, daß ein Pilz in einem Nährmedium kultiviert wird, das Maiskolben enthält.

2. Verfahren nach Anspruch 1, dadurch gekennzeichnet, daß als Pilz ein Thermomyces lanuginosus eingesetzt wird.

3. Verfahren nach einem der Ansprüche 1 oder 2, dadurch gekennzeichnet, daß als Pilz Thermomyces lanuginosus DSM 5826 eingesetzt wird.

4. Verfahren nach einem der Ansprüche 1 bis 3, dadurch gekennzeichnet, daß man den Pilz bei einer Temperatur von 30-70°C und bei einem pH-Wert von 5,0 bis 8,0 kultiviert.

5. Verfahren nach einem der Ansprüche 1 bis 4, dadurch gekennzeichnet, daß man den Pilz bei einer Temperatur von 45 bis 55°C und bei einem pH-Wert von 5,0 bis 8,0 kultiviert.

6. Verfahren nach Anspruch 3, dadurch gekennzeichnet, daß man Thermomyces lanuginosus DSM 5826 bei einer Temperatur von 45 bis 50 °C und bei einem pH-Wert von 6,0 bis 7,0 kultiviert.

7. Thermomyces lanuginosus DSM 5826.

8. Verfahren zur cellulasefreien Herstellung von Xylanase, dadurch gekennzeichnet, daß Thermomyces lanuginosus DSM 5826 in einem Nährmedium kultiviert wird.

9. Verwendung von Thermomyces lanuginosus DSM 5826 zur cellulasefreien Herstellung von Xylanase.

10. Verwendung von Thermomyces lanuginosus DSM 5826 zur enzymatischen Behandlung von xylan- und lignocellulosehältigen, pflanzlichen Rohstoffen und Faserstoffen aus solchen Rohstoffen.

## Claims

1. Process for the preparation of xylanase, characterised in that a fungus is cultivated in a nutrient medium which contains corn cobs.

2. Process according to Claim 1, characterised in that a Thermomyces lanuginosus is employed as fungus.

3. Process according to either of Claims 1 or 2, characterised in that Thermomyces lanuginosus DSM 5826 is employed as fungus.

4. Process according to any of Claims 1 to 3, characterised in that the fungus is cultivated at a temperature of 30-70°C and at a pH of 5.0 to 8.0.

5. Process according to any of Claims 1 to 4, characterised in that the fungus is cultivated at a temperature of 45 to 55°C and at a pH of 5.0 to 8.0.

6. Process according to Claim 3, characterised in that Thermomyces lanuginosus DSM 5826 is cultivated at a temperature of 45 to 50°C and at a pH of 6.0 to 7.0.

7. Thermomyces lanuginosus DSM 5826.

8. Process for the cellulase-free preparation of xylanase, characterised in that Thermomyces lanuginosus DSM 5826 is cultivated in a nutrient medium.

9. Use of Thermomyces lanuginosus DSM 5826 for the cellulase-free preparation of xylanase.

10. Use of Thermomyces lanuginosus DSM 5826 for the enzymatic treatment of xylan- and lignocellulose-containing vegetable raw materials and fibres composed of such raw materials.


**Revendications**

1. Procédé de préparation de xylanase, caractérisé en ce qu'on cultive un champignon dans un milieu nutritif renfermant des épis de maïs.

2. Procédé selon la revendication 1, caractérisé en ce qu'on utilise un thermomyces lanuginosus comme champignon.

3. Procédé selon l'une des revendications 1 ou 2, caractérisé en ce qu'on utilise un thermomyces lanuginosus DSM 5826 comme champignon.

4. Procédé selon l'une des revendications 1 à 3, caractérisé en ce qu'on cultive le champignon à une température de 30à 70°C à une valeur de pH de 5,0 à 8,0.

5. Procédé selon l'une des revendications 1 à 4, caractérisé en ce qu'on cultive le champignon à une température de 45 à 55°C et à une valeur de pH de 5,0 à 8,0.

6. Procédé selon la revendication 3, caractérisé en ce qu'on cultive le thermomyces lanuginosus DSM 5826 à une température de 45 à 50°C et à une valeur de pH de 6,0 à 7,0.

7. Thermomyces lanuginosus DSM 5826

8. Procédé de préparation de xylanase exempte de cellulase, caractérisé en ce qu'on cultive le thermomyces lanuginosus DSM 5826 dans un milieu nutritif.

9. Utilisation du thermomyces lanuginosus DSM 5826 à la préparation de xylanase exempte de cellulase.

10. Utilisation de thermomyces lanuginosus DSM 5826 pour le traitement enzymatique de matières brutes de plantes renfermant du xylane et de la lignocellulose et les matières fibreuses de telles matières brutes.

Fig. 1

Fig. 2

Fig. 3

Fig. 4